# EUROPEAN PATENT APPLICATION

(11) **EP 1 662 239 A2**
(43) Date of publication of application: **31.05.2006**
(21) Application number: 05022807.1
(22) Date of filing: 19.10.2005
(51) Int. Cl.: G01K 13/00, G01K 7/02

(54) **Electronic clinical thermomether**

(30) Priority: 20.10.2004 JP 2004305676
(71) Applicant: Omron Healthcare Co., Ltd., Kyoto 615-0084 (JP)
(72) Inventor: Sato, Taiga c/o Omron Healthcare Co.,Ltd., Ukyo-ku,Kyoto-shi Kyoto 615-0084 (JP); Nakada, Tetsuya c/o Omron Healthcare Co.,Ltd., Ukyo-ku,Kyoto-shi Kyoto 615-0084 (JP)
(74) Representative: Kilian, Helmut

(57) **Abstract**

In turn-on state, it is monitored whether a power switch 15 is pressed or not (ST1). When not pressed, it is continuously monitored whether pressed or not. When it is judged that the power switch 15 is pressed, it is monitored whether the power switch 15 is pressed continuously for no less than 2 seconds or not (ST2). When it is judged that the power switch 15 is not pressed continuously for no less than 2 seconds, back to ST1, it is continuously monitored whether the power switch 15 is pressed or not. When it is judged that the power switch 15 is pressed continuously for no less than 2 seconds, the process of turning off the power is started (ST3).

## Description

### Background of the Invention

### Field of the Invention

The present invention relates to an electronic clinical thermometer, more particularly to prevention of misoperation during measurement.

### Description of the Related Art

Various electronic clinical thermometers for measuring the body temperature are proposed, including the contact type of measuring the body temperature by keeping the temperature sensing unit in contact with the measuring area (beneath the armpit, under the tongue, etc.), and the contact-free type of measuring the heat radiation from the tympanic membrane, etc. A general contact type electronic clinical thermometer comprises a slender main body shaped like a pen, a temperature sensing unit provided at the tip of the main body, a display unit for showing the result of measurement provided at a side of the main body, a power switch provided in the rear part or side of the main body, and a control unit for executing measurement and arithmetic processing built in the main body.

In such electronic clinical thermometer, the most popular measuring area is beneath the armpit, and the user inserts the electronic clinical thermometer beneath the armpit inside the clothes, and holds for scores of seconds to several minutes to measure. The user must hold the electronic clinical thermometer beneath the armpit in contact at a position not seen from above the clothes, and in particular children tend to change the position during measurement, happening to press the power switch by mistake to cut off the power.

In such background, it is proposed to support the push type switch button by the back lid formed at other end of the bar-like case, so that the switch position may not coincide with the finger position when holding the electronic clinical thermometer (refer to, for example, Japanese Patent Application Publication No. 6-29790). In another proposal, the electronic clinical thermometer has no switch to be manipulated by the user, in which the electronic clinical thermometer having a built-in magnet lead switch is stored in an electronic clinical thermometer case having a magnet for actuating the magnet lead switch (refer to, for example, Japanese Utility Model Application Publication No. 7-44986).

However, in the art disclosed in Japanese Patent Application Publication No. 6-29790, misoperation can be avoided when holding the electronic clinical thermometer by fingers and inserting or taking out from beneath the armpit, but the switch button may be touched by mistake and the power is turned off during measurement inside the clothes.

In the art disclosed in Japanese Utility Model Application Publication No. 7-44986, there is no switch to be manipulated by user, and power turn-off by misoperation during measurement is avoided, but the structure of electronic clinical thermometer is complicated, and the power source of the electronic clinical thermometer cannot be turned off if the case is not at hand.

### Summary of the Invention

The invention is based on the problems of these prior arts, and it is an obj ect thereof to prevent power turn-off by misoperation during measurement of body temperature in unseen state without requiring complicated structure.

An electronic clinical thermometer in one aspect of the invention comprises a power switch manipulated by the user to turn off the power from power-on state, and a control unit for turning off the power according to the manipulation of the power switch, in which the control unit turns off the power when it is judged that the power switch is manipulated in a predetermined pattern for turning off the power.

Preferably, the power switch also serves as manipulation switch for turning on the power by the user, and the manipulation pattern for turning on the power is a pattern different from a predetermined pattern for turning off the power.

Preferably, the predetermined pattern for turning off the power is to press the power switch continuously no less than a specified time.

Preferably, it further comprises an alarm unit for issuing an alarm sound, and the control unit drives and controls the alarm unit for issuing an alarm sound and turns off the power when it is judged that the power switch is pressed continuously no less than a specified time as predetermined pattern for turning off the power.

Preferably, the predetermined pattern for turning off the power is to press the power switch continuously no less than a first specified time and within a second specified time.

Preferably, it further comprises an alarm unit for issuing an alarm sound, and the control unit drives and controls the alarm unit for issuing an alarm sound when it is judged that the power switch is pressed continuously no less than a first specified time as predetermined pattern for turning off the power, and turns off the power when it is judged that the power switch is released within a second specified time after issue of the alarm sound.

Preferably, the predetermined pattern for turning off the power is to press the power switch by a specified number of times within a specified time.

According to the invention, by turning off the power only when the power switch is manipulated in a predetermined characteristic manipulation pattern, it is possible to prevent power interruption during body temperature measurement due to accidental contact with the power switch and the like. By noticing by the alarm sound when the predetermined manipulation pattern is securely executed, the user can manipulate securely for turning off the power.

### BRIEF DESCRIPTION OF THE DRAWINGS

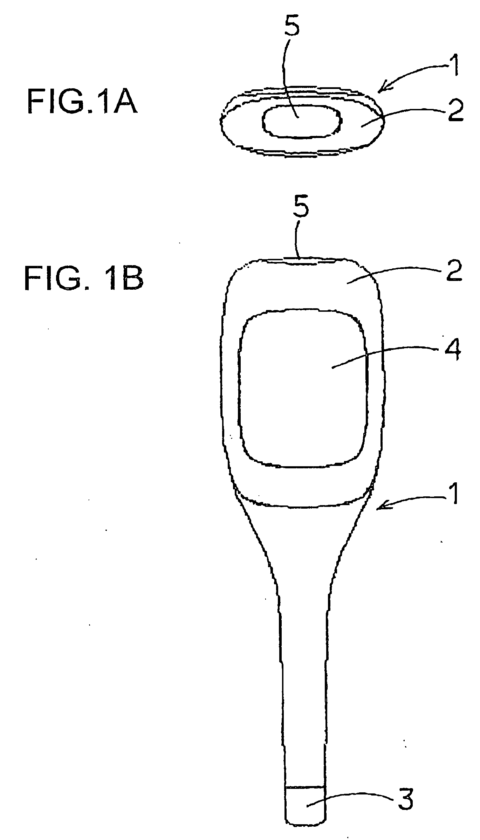

[0047] FIG. 1 is an outline drawing of electronic clinical thermometer in an embodiment of the invention, FIG. 1A is a top view, and FIG. 1B is a front view;
FIG. 2 is a function block diagram of electronic clinical thermometer of the embodiment;
FIG. 3 is a flowchart for explaining the power turn-off process of electronic clinical thermometer of the embodiment;
FIG. 4 is a flowchart for explaining the power turn-off process of electronic clinical thermometer in other embodiment of the invention; and
FIG. 5 is a flowchart for explaining the power turn-off process of electronic clinical thermometer in another embodiment of the invention.

### Detailed Description of the Preferred Embodiments

Preferred embodiments of the invention are described below while referring to the drawings.

(Outline structure of electronic clinical thermometer)
FIG. 1 shows an outline structure of electronic clinical thermometer in the present embodiment. FIG. 1A is a top view of the electronic clinical thermometer and FIG. 1B is a front view thereof.

The electronic clinical thermometer 1 of the embodiment has a proper shape and size for contacting with the measuring area such as beneath the armpit of the user. The electronic clinical thermometer 1 comprises a temperature sensing unit 3, a display unit 4, and a power switch manipulation unit 5, which are provided in a main body 2.

The main body 2 is made of plastics, having a slender shape in the lower part to be inserted easily by the user beneath the armpit and so on, and a wide rectangular shape in the upper part for displaying the result of measurement and so on largely and realizing an excellent visual recognition.

The temperature sensing unit 3 is made of stainless steel, and is provided at the lower end of the main body 2, and a thermistor (described below) is incorporated inside as temperature sensing element.

The display unit 4 is to show the result of measurement of body temperature or state of operation and so on, and it is provided at the upper front side of the main body 2.

The power switch manipulation unit 5 is for manipulating the power switch (described below) built in the main body 2, and it is provided at the upper end of the main body 2.

(Function blocks of electronic clinical thermometer)

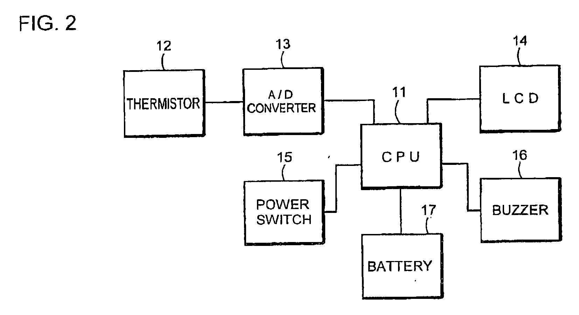

FIG. 2 shows function block diagram of electronic clinical thermometer of the embodiment.

The electronic clinical thermometer 1 of the embodiment has a processing circuit incorporated in the inside of the main body 2. The processing circuit is built, for example, in the temperature sensing unit 3, and includes a thermistor 12 for converting the user's body temperature into an analog electrical signal, an A/D converter 13 for converting the analog electrical signal from the thermistor 12 into a digital signal, a CPU 11 as control unit for receiving the digital signal from the A/D converter 13 and on/off signal from the power switch 15, and performing arithmetic operations for measuring the body temperature and turning on or off the power, an LCD 14 as a display unit for showing the result of body temperature measurement or state of operation, a power switch 15 for receiving power on/off command, a buzzer 16 as alarm unit for issuing an alarm sound, and a battery 17 as power source.

(Body temperature measuring operation of processing circuit)
Body temperature measuring operation of processing circuit of the electronic clinical thermometer 1 of the embodiment is realized by a known art, and is executed, for example, as follows.

Before the power is turned on, the user presses down the power switch manipulation unit 5 provided at the upper end of the main body 2, then the power switch 15 in the processing circuit is manipulated, and the power of the processing circuit is turned on. The CPU 11 executes an initialization, and tells it is ready to start measurement of body temperature by displaying in the LCD 14. When the user brings the temperature sensing unit 3 into contact with the measuring area beneath the armpit and so on, the thermistor 12 in the temperature sensing unit 3 converts the user' s body temperature information into an analog electrical signal. The analog electrical signal from the thermistor 12 is converted into a digital signal by the A/D converter 13, and put into the CPU 11. On the basis of the entered digital signal, the CPU 11 judges that body temperature measurement is started, displays that measurement is in process in the LCD 14. Also, on the basis of the entered digital signal, the CPU 11 calculates the body temperature of the user. When calculation of body temperature is over, the CPU 11 notices completion of measurement by the LCD 14 and buzzer 16, and displays the result of measurement of body temperature in the LCD 14. The user recognizes completion of body temperature measurement by the alarm sound of the buzzer 16, then takes out the electronic clinical thermometer 1 from the measuring area, and confirms the result of measurement displayed in the LCD 14, and presses down the power switch manipulation unit 5. When the power switch 15 is manipulated, the CPU 11 turns off the power of the processing circuit, and the measuring operation is completed.

When the user measures the body temperature beneath the armpit, generally, without taking off the clothes, the electronic clinical thermometer 1 is inserted inside of the clothes, until the temperature sensing unit 3 touches beneath the armpit. This state must be maintained for scores of seconds to several minutes to measure the body temperature. The user maintains the measuring position by pressing the electronic clinical thermometer 1 beneath the armpit, or supporting the electronic clinical thermometer 1 by hand from above the clothes. However, children often change the position or move the supporting hand, and the power switch manipulation unit 5 may be pressed by mistake.

(Power turn-off process of electronic clinical thermometer)
In the electronic clinical thermometer 1 of the embodiment, the CPU 11 is designed to execute the power turn-off process only when it is judged that the power switch 15 is manipulated in a predetermined pattern for turning off the power from the turn-on state.

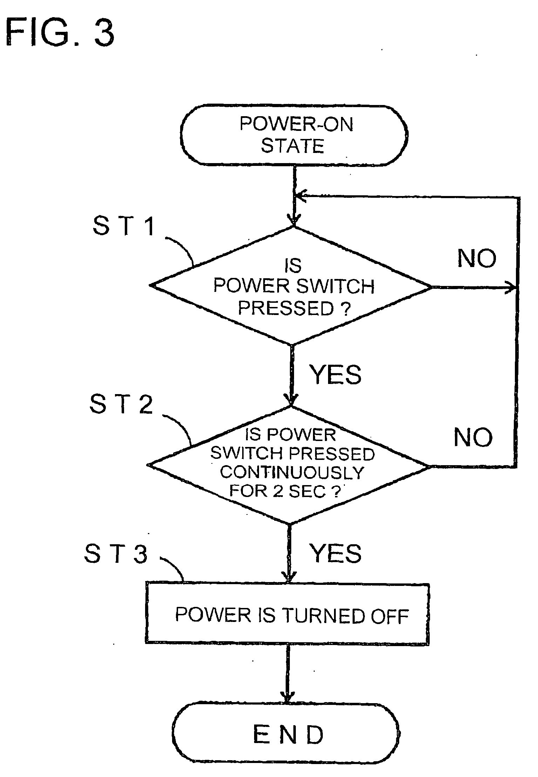

FIG. 3 is a flowchart for explaining the power turn-off process of the electronic clinical thermometer 1 of the embodiment.

While the processing circuit of the electronic clinical thermometer 1 is in turn-on state, the CPU 11 monitors whether the power switch 15 is pressed or not (step 1, hereinafter referred to as ST1): When it is judged that the power switch 15 is not pressed (NO at ST1) , the CPU 11 returns to ST1, and continues to monitor whether the power switch 15 is pressed or not. When it is judged that the power switch 15 is pressed (YES at ST1), the CPU 11 monitors whether the power switch 15 is pressed continuously for no less than 2 seconds or not (ST2) . When it is judged that the power switch 15 is not pressed continuously for no less than 2 seconds (NO at ST2), the CPU 11 returns to ST1, and continues to monitor whether the power switch 15 is pressed or not. When it is judged that the power switch 15 is pressed continuously for no less than 2 seconds (YES at ST2), the CPU 11 starts the process of turning off the power of the processing circuit (ST3), and the process is terminated.

In this manner, if accidentally touching the power switch manipulation unit 5 while measuring the body temperature by the user by inserting the electronic clinical thermometer 1 beneath the armpit inside of the clothes, the power of the processing circuit is not turned off, and measurement of body temperature is not interrupted.

In this embodiment, the specified time for pressing the power switch 15 continuously at ST2 is no less than 2 seconds, and this duration is determined in consideration that power turn-of f process is not executed if the hand or other part of the body accidentally and momentarily touches the power switch operation unit 5, and that the operability is worsened by demanding the user to press the power switch manipulation unit 5 continuously longer than necessary. If this specified time is set preliminarily, however, it is not limited to 2 seconds.

In the electronic clinical thermometer 1 of the embodiment, the power switch 15 is designed to be used not only when turning off the power of the processing circuit, but also when turning on. When turning on the power, body temperature measurement is not started naturally, and the user can manipulate the power switch manipulation unit 5 while recognizing visually, and it may be designed to turn on the power only when the power switch 15 is merely pressed down. Thus, by setting the manipulation pattern of the power switch manipulation unit 5 differently between turn-on operation and turn-off operation, the power turn-off operation by the user is more reliable.

At ST3, when the CPU 11 starts the power turn-off process, it may drive the buzzer 16 to issue an alarm sound. Thus, the user easily recognizes that the power turn-on off operation is established, and if the power turn-off process is executed by misoperation during measurement of body temperature, interruption of body temperature measurement is readily recognized.

Other example of power turn-off process of the electronic clinical thermometer is explained below as other embodiment of the invention.

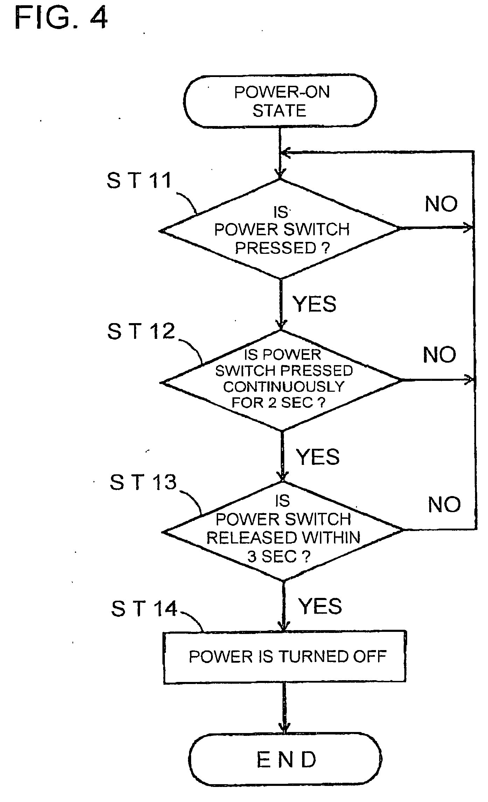

FIG. 4 is a flowchart for explaining the power turn-off process of the electronic clinical thermometer 1 of this embodiment.

While the processing circuit of the electronic clinical thermometer 1 is in turn-on state, the CPU 11 monitors whether the power switch 15 is pressed or not (ST11). When it is judged that the power switch 15 is not pressed (NO at ST11), the CPU 11 returns to ST11, and continues to monitor whether the power switch 15 is pressed or not. When it is judged that the power switch 15 is pressed (YES at ST11) , the CPU 11 monitors whether the power switch 15 is pressed continuously for no less than 2 seconds or not (ST12). When it is judged that the power switch 15 is not pressed continuously for no less than 2 seconds (NO at ST12) , the CPU 11 returns to ST11, and continues to monitor whether the power switch 15 is pressed or not. When it is judged that the power switch 15 is pressed continuously for no less than 2 seconds (YES at ST12) , the CPU 11 monitors whether the power switch 15 is released from pressing within the next 3 seconds or not (ST13) . If it is judged that the power switch 15 is not released from pressing within the next 3 seconds (NO at ST13) , the CPU 11 returns to ST11, and continues to monitor whether the power switch 15 is pressed or not. When it is judged that the power switch 15 is released from pressing within the next 3 seconds (YES at ST13) , the CPU 11 executes the process of turning off the power of the processing circuit (ST14) , and the process is terminated.

In this embodiment, if the power switch manipulation unit 5 is pressed for a long time accidentally while measuring the body temperature by the user by inserting the electronic clinical thermometer 1 beneath the armpit inside of the clothes, the power of the processing circuit is not turned off, and measurement of body temperature is not interrupted.

At ST12, when the CPU 11 judges that the power switch 15 is pressed continuously for 2 seconds, it may drive the buzzer 16 to issue an alarm sound. Thus, the user easily recognizes the timing of releasing the power switch manipulation unit 5 from pressing for power turn-off operation, and power turn-off operation can be executed more securely. In case the power switch 15 is pressed no less than 2 seconds by mistake due to misoperation during measurement of body temperature, the power turn-off operation is not executed if the power switch 15 is pressed continuously over 3 seconds after issue of the alarm sound by the buzzer 16, so that interruption of body temperature measurement is prevented.

In this embodiment, too, as far as the predetermined time is preset at ST12 and ST13, the duration is not limited to the time specified in the embodiment.

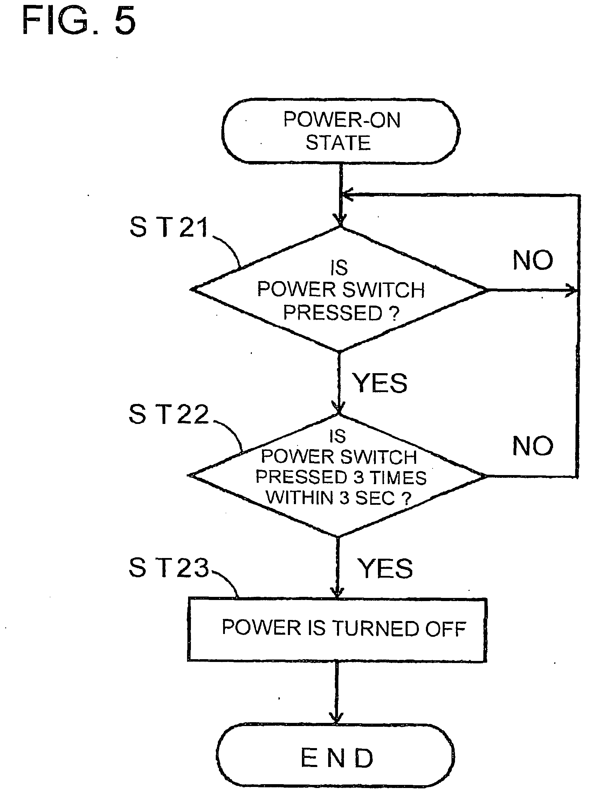

FIG. 5 is a flowchart for explaining the power turn-off process of the electronic clinical thermometer 1 of another embodiment.

While the processing circuit of the electronic clinical thermometer 1 is in turn-on state, the CPU 11 monitors whether the power switch 15 is pressed or not (ST21). When it is judged that the power switch 15 is not pressed (NO at ST21), the CPU 11 returns to ST21, and continues to monitor whether the power switch 15 is pressed or not. When it is judged that the power switch 15 is pressed (YES at ST21), the CPU 11 monitors whether the power switch 15 is pressed three times within next 3 seconds or not (ST22). When it is judged that the power switch 15 is not pressed three times within next 3 seconds (NO at ST22) , the CPU 11 returns to ST21, and continues to monitor whether the power switch 15 is pressed or not. When it is judged that the power switch 15 is pressed three times within next 3 seconds (YES at ST22), the CPU 11 starts the process of turning-off the power of the processing circuit (ST23), and the process is terminated.

In this embodiment, if the power switch manipulation unit 5 is touched accidentally while measuring the body temperature by the user by inserting the electronic clinical thermometer 1 beneath the armpit inside of the clothes, the power of the processing circuit is not turned off, and measurement of body temperature is not interrupted.

In this embodiment, too, the specified duration at ST22 and the specified number of times of pressing the power switch 15 are not limited to the time specified in the embodiment as far as they are preset.

At ST23, when the CPU 11 starts power turn-off process, it may drive the buzzer 16 to issue an alarm sound. Thus, the user easily recognizes the establishment of power turn-off operation, and if power turn-off process is executed by misoperation during measurement of body temperature, interruption of body temperature measurement is readily recognized.

All the illustrated embodiments disclosed herein should be considered as examples in all respects and not limitative. The scope of the invention is not limited to the above explanation, but should be understood to include all changes and modifications that fall within metes and bounds of the claims, or equivalence of such metes and bounds thereof are therefore intended to be embraced by the claims.

## Claims

1. An electronic clinical thermometer comprising a power switch manipulated by the user to turn off the power from power-on state, and a control unit for turning off the power according to the manipulation of the power switch,
wherein the control unit turns off the power when it is judged that the power switch is manipulated in a predetermined pattern for turning off the power.

2. An electronic clinical thermometer according to claim 1, wherein the power switch also serves as manipulation switch for turning on the power by the user, and the manipulation pattern for turning on the power is a pattern different from a predetermined pattern for turning off the power.

3. An electronic clinical thermometer according to claim 1 or 2, wherein the predetermined pattern for turning off the power is to press the power switch continuously no less than a specified time.

4. An electronic clinical thermometer according to claim 3, further comprising an alarm unit for issuing an alarm sound,
wherein the control unit drives and controls the alarm unit for issuing an alarm sound and turns off the power when it is judged that the power switch is pressed continuously no less than a specified time as predetermined pattern for turning off the power.

5. An electronic clinical thermometer according to claim 1 or 2, wherein the predetermined pattern for turning off the power is to press the power switch continuously no less than a first specified time and within a second specified time.

6. An electronic clinical thermometer according to claim 1 or 2, further comprising an alarm unit for issuing an alarm sound,
wherein the control unit drives and controls the alarm unit for issuing an alarm sound when it is judged that the power switch is pressed continuously no less than a first specified time as predetermined pattern for turning off the power, and turns off the power when it is judged that the power switch is released within a second specified time after issue of the alarm sound.

7. An electronic clinical thermometer according to claim 1 or 2, wherein the predetermined pattern for turning off the power is to press the power switch by a specified number of times within a specified time.
